# EUROPEAN PATENT SPECIFICATION

(11) **EP 3 669 338 B1**
(45) Date of publication and mention of the grant of the patent: **24.04.2024**
(21) Application number: 18846598.3
(22) Date of filing: 15.08.2018
(51) Int. Cl.: G08B 21/04, G08B 3/10, G06Q 50/06, G08B 21/20

(54) **PASSIVE CARE CONTROL METHOD AND ASSOCIATED SYSTEMS**
VERFAHREN ZUR STEUERUNG VON PASSIVER PFLEGE UND ZUGEHÖRIGE SYSTEME
PROCÉDÉ DE COMMANDE DE SOINS PASSIVE ET SYSTÈMES ASSOCIÉS

(30) Priority: 16.08.2017 AU 2017903297
(43) Date of publication of application: 24.06.2020
(73) Proprietor: Caroma Industries Limited, Queensland, 4006 (AU); micas AG, 09376 Oelsnitz/Erzgebirge (DE)
(72) Inventor: CUMMINGS, Stephen John, East Mona Vale, New South Wales 2103 (AU)
(74) Representative: Steiniger, Carmen
(86) International application number: PCT/AU2018/000137
(87) International publication number: WO 2019/033144

(56) References cited:
- WO-A1-2015/124972
- WO-A2-2013/049748
- US-A1- 2012 215 369
- US-A1- 2012 330 615
- US-A1- 2016 005 015
- US-A1- 2016 041 565
- US-A1- 2016 350 880
- US-A1- 2016 350 880
- US-A1- 2017 175 367

## Description

### Technical Field

The present invention relates generally to a passive care control method and associated systems.

### Background

In certain facilities where people live who require supervision or monitoring for health or care reasons, when such a person becomes incapacitated and requires assistance, it is not always easy for that person to indicate to a relevant authority or authorised carer that assistance is required. For example, in healthcare facilities and aged care facilities etc., providing specialised equipment such as alert systems and alarm buttons that have been specifically developed to enable these persons to only request assistance in times of need may not be viable cost wise and may also require additional installation costs.

In an aged car facility for example, residents may live semi-autonomously in their own unit. Many accidents occur in wet environments such as the bathroom. However, when an accident occurs, it is not unusual for the resident to be incapacitated and not be in a position to call out for help. Similar scenarios are also envisioned in other environments where other physical consumables are provided, such as gas and electricity.

In document US 2016/0350880 A1 a system for sensing water device runtime is disclosed, in which sensors are used to measure each interaction and/or water run time of a water use appliance. A system of node units receives the measured data packages and forwards them to a central internet gateway which uploads the data packets to a cloud based database which analyses and reports the information. Document US 2016/041565 discloses systems, methods, and apparatus configured to automatically monitor water characteristics and control water flow through a plumbing system.

### Summary

It is an object of the present invention to substantially overcome, or at least ameliorate, one or more disadvantages of existing arrangements.

Disclosed are arrangements which seek to address the above problems by monitoring usage of a physical consumable and determining whether abnormal usage of the physical consumable has occurred. Upon determination of abnormal usage, an abnormal usage indicator may be generated and sent to a registered device to provide an alert of the abnormal usage.

The invention is defined by the independent claims and set out in the appended set of claims.

According to a first aspect of the present disclosure, there is provided a method for detecting abnormal usage of water in a water usage environment, wherein the method comprises the steps of monitoring detected usage data associated with the provision of water in a water usage environment using at least one water provisioning device that is arranged to provide the water; and using a water monitoring device which is part of or integrated with one of the at least one water provisioning devices; and detecting abnormal usage of the water based on the monitoring of the detected usage data, wherein the detecting of the abnormal usage is performed by the water monitoring device associated with the water provisioning device, wherein the method further comprising the steps of comparing the detected usage data with prior usage data associated with prior use of the water by the water provisioning device, and detecting the abnormal usage of the water based on the comparison of the detected usage data with the prior usage data, wherein the step of comparing the detected usage data with the prior usage data comprises detecting whether the detected usage data matches a usage pattern associated with the prior usage data or wherein the step of comparing the detected usage data with the prior usage data comprises detecting a maximum and/or minimum prior usage value in the prior usage data and comparing the detected maximum and/or minimum prior usage value with maximum and/or minimum values in the detected usage data.

The method may include the step of generating an alert based on the detection of the abnormal usage.

The method may include the step of sending the generated alert to one or more registered devices associated with the usage of the physical consumable.

A water monitoring device is provided, the water monitoring device comprising I/ O port, transceiver module , a power unit, a processor and memory, wherein the I/O port connects to a sensor, wherein the water monitoring device is part of or integrated with a water provisioning device arranged to provide water and the memory comprises software code arranged to cause the processor to operate according to defined instructions to implement the method according to the previously described first aspect of the invention.

The water monitoring device may be part of or integrated with one or more of an item of tap ware, a shower unit and a flush unit.

A building management system comprising a central gateway device is provided, wherein the central gateway device includes a power unit, a transceiver module , a processor and memory, wherein the transceiver module connects and communicates data with one or more water monitoring devices.

A system comprising a building management system and a server is provided, wherein the server comprises a power unit, an input module, an output module, a processor and memory, wherein the input module of the server connects and receives data from the central gateway device.

### Brief Description of the Drawings

At least one embodiment of the present invention will now be described with reference to the drawings and appendices, in which:
Fig. 1 shows components forming at least part of a building management system for monitoring abnormal usage of water according to the present disclosure;
Fig. 2 shows a water monitoring device according to the present disclosure;
Fig. 3 shows a gateway device forming at least part of a building management system according to the present disclosure;
Fig. 4 shows a server forming at least part of a building management system according to the present disclosure;

### Detailed Description including Best Mode

The herein description provides details of a method, system, and associated components that provide passive care to one or more individuals. The term "passive" is intended to mean that care is provided in the background without the user having to actively indicate that care is specifically required. That is, a situation is passively monitored without the user being required to diverge from their everyday routine.

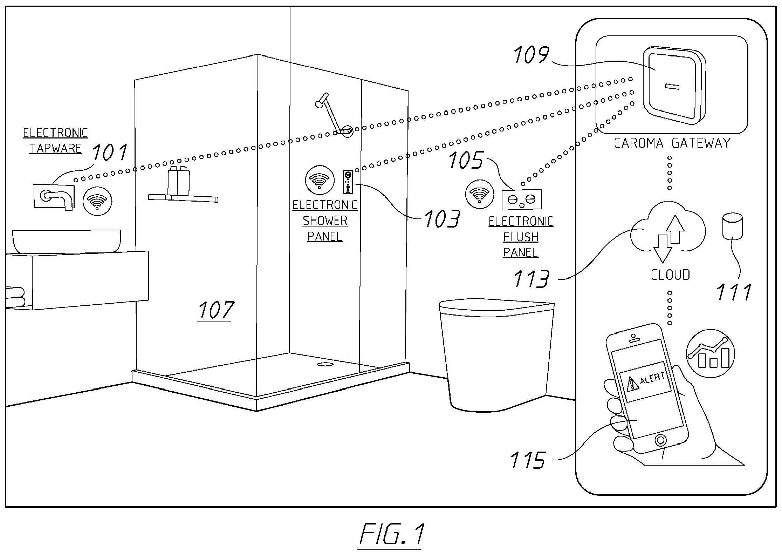

Fig. 1 shows an example arrangement of various electronic control devices in the form of water provisioning devices, such as electronic tap ware 101, an electronic shower unit 103 and an electronic toilet flush 105, which are used in a physical consumable usage environment, such as a bathroom 107.

Each of these electronic control devices includes an electronic system that is arranged to detect and/or monitor usage of the water that the device is using in order to generate usage data associated with that device. One or more of the electronic control devices may be part of a building management system (BMS).

The electronic system in each electronic control device is also arranged to wirelessly communicate with a central gateway device 109. For example, the electronic control device may wirelessly communicate using Bluetooth or a Wi-Fi connection. Each electronic control device may have a unique ID in order for the central gateway device to associate the received data with that electronic control device.

The central gateway device is arranged to communicate with a server 111 via the Internet 113. The central gateway device may be part of a building management system (BMS). The central gateway device may connect with multiple electronic control devices throughout a single building, for example, in multiple bathrooms in a single aged care facility. There may also be multiple central gateway devices that are used where each connects to a group of electronic control devices located in one or more groups of bathrooms.

The central gateway device is connected to the Internet (e.g. via a local wireless modem built into the device, a local independent wirelessly connected modem, a 3G/4G data enabled connection (e.g. via a dongle or the like, via a wired connection to a modem, or via an ad-hoc connection (e.g. Bluetooth) to a smart telephone).

The server 111 is arranged to communicate with one or more registered devices 115, which may be, for example, in the form of a smart phone, a laptop, tablet, PC or any other consumable electronic device that can communicate with the server.

The devices may be registered by providing details associated with the device to a server via a website. The details may include the number of the device (if a telephone), IP address or location, ID number etc. Further, when registering the device, details of the user of the consumable provisioning device may be provided, such as the user's location, address, name, contact number etc. Further, when registering the device, details of the consumable provisioning device may also be provided, such as the device's unique ID number, location, type etc. This information is stored by the server to enable any alerts or alarms associated with a user or consumable provisioning device to be communicated to the correct registered device.

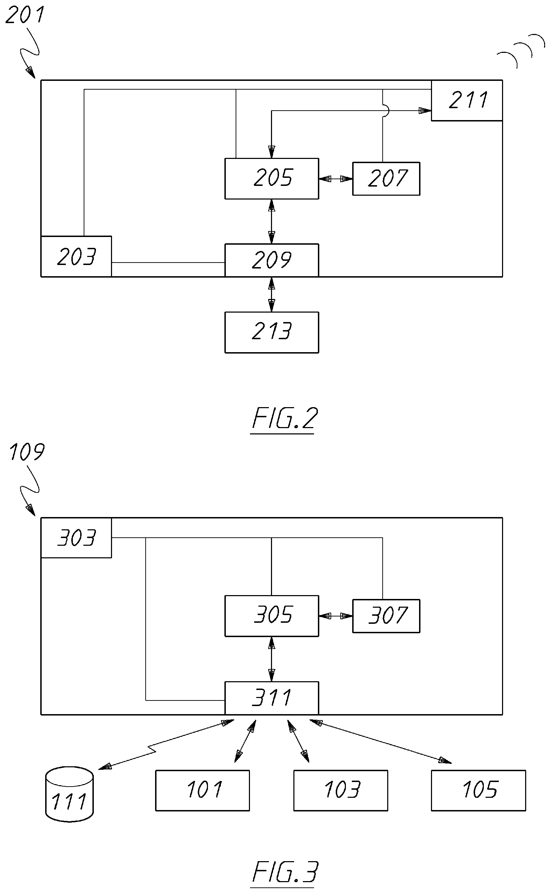

Details of an example water monitoring device incorporated within or in communication with the water provisioning device is provided in Fig. 2

Fig. 2 shows a block diagram of a physical consumable monitoring device 201 which forms part of or is integrated into an electronic control device (e.g. 102, 103, 105) for controlling operation of one or more bathroom products.

The monitoring device 201 has a power unit 203 that provides power to a processor 205, memory 207, I/O port 209 and transceiver module 211.

The memory 207 stores an algorithm in the form of a software program to cause the processor 205 to operate according to defined instructions. The memory 207 may also be used to store (temporarily or semi-permanently) data associated with the operations of the processor 205. This data may include usage data as well as indications of abnormal usage associated with one or more bathroom products.

The control device may be connected to one or more taps, shower units and toilet flush units as well as any other type of bathroom product that uses water. The control device may be used by a user to control the use of the bathroom product using control signals, such as switching the flow of water in the bathroom product on and off etc. The monitoring device is arranged to monitor the provision of water. That is, usage data for the provision of the physical consumable may be monitored and recorded using the monitoring device. It will be understood that the components in the monitoring device may be separate components to those in the control device or may be shared (i.e. the same) components used in the control device.

In one example described in more detail herein, the software in the monitoring device is arranged to cause the processor to monitor the provision (and so usage) of the water (physical consumable) for a particular bathroom product associated with a particular user. In this example, the software in the monitoring device is arranged to cause the processor to monitor (and so determine) whether the provision of the water is abnormal usage. Various methods may be employed to detect whether abnormal usage has occurred, and these are discussed in detail herein.

In a non claimed example, the monitoring device may communicate with the electronic control device to obtain usage data of the water in order to determine whether the provision of the water is abnormal usage.

The input port 209 connects to a sensor 213 that senses usage of the physical consumable, such as water. Any known sensor or sensing system may be used to detect the provision of the physical consumable. The input port receives sensor data from the sensor, where the sensor data is based on the usage of the consumable. Further, the input port 209 may be part of a general I/O port that includes an output to communicate one or more control signals to control how the physical consumable is provided by the provisioning device. For example, the control signals may determine whether water is provided, how much water is provided, the amount of water flow etc.

The transceiver module 211 connects and communicates data with the central gateway device 109. This data may include one or more of usage data, abnormal usage data, alert data, alarm data, or any other data that has been generated or obtained by the electronic control device and/or monitoring device.

It will be understood that according to a non claimed example the physical consumable monitoring device may be a physically separate device from the electronic control device but may still communicate with the electronic control device to obtain usage data of a physical consumable associated with a particular provisioning device and/or a particular user.

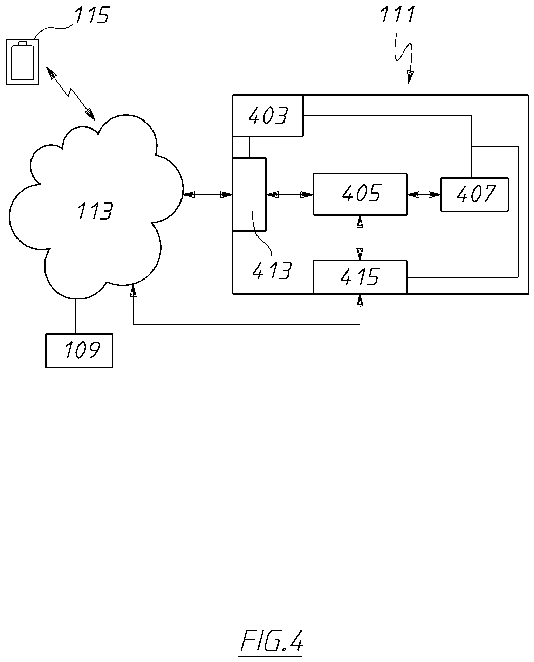

An example central gateway device and an example server are provided in Figs. 3 and 4 respectively.

Fig. 3 shows a block diagram of a central gateway device 109. The central gateway device may be part of a building management system (BMS).

The central gateway device 109 has a power unit 303 that provides power to a processor 305, memory 307 and a transceiver module 311.

The memory 307 stores an algorithm in the form of a software program to cause the processor 305 to operate according to defined instructions. The memory 307 may also be used to store (temporarily or semi-permanently) data associated with the operations of the processor 305. This data may include usage data as well as indications of abnormal usage associated with one or more bathroom products.

The transceiver module 311 connects and communicates data with one or more physical consumable monitoring devices 201 and/or electronic control devices (101, 103 or 105). This data may include one or more of usage data, abnormal usage data, alert data, alarm data, or any other data that has been generated or obtained by the central gateway device 109.

The transceiver module 311 also connects and communicates data with one or more servers 111. This data may also include one or more of usage data, abnormal usage data, alert data, alarm data, or any other data that has been generated or obtained by the central gateway device 109. The communication with the server may be via a modem or any other suitable communication means to enable the central gateway device to communicate with the server. For example, the central gateway device may communicate with the server via the Internet using any suitable communication protocols or messages.

Fig 4 shows a block diagram of a server 111. The server may be part of a building management system (BMS).

The server 111 has a power unit 403 that provides power to a processor 405, memory 407, an input module 413 and an output module 415.

The memory 407 stores an algorithm in the form of a software program to cause the processor 405 to operate according to defined instructions. The memory 407 may also be used to store (temporarily or semi-permanently) data associated with the operations of the processor 405. This data may include usage data as well as indications of abnormal usage associated with one or more bathroom products. The memory 407 may be used in conjunction with other storage modules (not shown) and databases.

The input module 413 of the server 111 connects and receives data from one or more central gateway devices 109 either directly or via a building management system. This data may include one or more of usage data, abnormal usage data, alert data, alarm data, or any other data that has been generated or obtained by the central gateway device 109, a connected physical consumable monitoring device 201 or an electronic control device (101, 103 or 105). The communication between the server and the central gateway device may utilise any suitable form of communication protocols to enable the server to communicate with the central gateway device. For example, the server may communicate with the central gateway device via the Internet using any suitable communication protocols or messages.

The output module 415 of the server 111 connects and communicates data to one or more registered devices 115. This data may include one or more of usage data, abnormal usage data, alert data, alarm data, or any other data that has been generated or obtained by the server 111. The communication with the server may be via an output port or any other suitable communication device to enable the server to communicate with the registered devices. For example, the server may communicate with the registered devices via the Internet using any suitable communication protocols or messages.

The various components described above in relation to Figs. 1-4 are used to monitor the provision of water. The provision of the physical consumable is monitored to assess or determine whether abnormal usage has occurred that would indicate that there is a risk that the associated user requires assistance. For example, the current usage may be compared to known potential alert usage patterns for a particular consumable provisioning device and/or a particular user. Also, prior usage may be recorded to assess whether abnormal usage is occurring. If it is determined that abnormal usage is occurring, then an abnormal usage notification in the form of, for example, an alert and/or alarm may be generated and sent to one or more registered devices that are associated with the user or usage of the consumable. The alert or alarm may be, for example, one or more of an email, an SMS, a telephone calls, an electronic alert, a communication to a software program operating on an electronic device etc. Any of the physical consumable monitoring device, central gateway device and server may i) determine whether abnormal usage has occurred, ii) communicate that abnormal usage has occurred, iii) generate an alert or alarm, and iv) communicate an alert or alarm.

Normal usage behaviour patterns may be recorded by the system. That is, metrics associated with normal usage of the various products (e.g. bathroom products) when a person is not incapacitated may be tracked and recorded. Using artificial intelligence (Al), usage patterns over a defined period of time, e.g. a month, may be recorded and associated with an individual user, who is associated with the products. For example, if a user uses a particular bathroom product at the same time every day, this pattern is recorded. If the detected current usage (or non-usage) by the user deviates from this pattern, an alert may be generated. Patterns of usage of one product may be monitored in association with patterns of usage of other products in order to provide a more complex analysis. For example, shower usage may be monitored alongside toilet usage in order to detect abnormal usage. A series of weightings may be applied to different products based on whether it would be considered essential for their use within a defined period, such as the toilet for example. Whereas, a lower weighting may be applied to usage of the shower or tap ware. That is, usage of what are considered more essential products may have higher weighting values associated with them in order to determine whether abnormal usage has occurred.

Several examples are now provided to show how the herein described system, or variations of it, may operate in order to detect abnormal usage of a physical consumable.

The following example describes how a water monitoring device may detect abnormal usage in a bathroom environment.

The water monitoring device may be associated with, part of, or integrated with one or more physical consumable provisioning devices and/or electronic control devices. The physical consumable provisioning devices is part of, or integrated with one or more electronic control devices.

The electronic control devices may be a device such as a tap ware device 101, shower unit 103 or flush unit 105 as shown in Figure 1. The functionality of the monitoring device may be incorporated into the existing components of the control device or maybe formed as separate components.

Where the electronic control device is a shower unit 103, for example, the control device detects usage of water provisioned by the shower unit to generate usage data. The usage data may be recorded in memory by the control device alongside a unique ID associated with the shower unit. The usage data may be recorded in memory by the control device alongside a unique ID associated with a user associated with the shower unit. The usage data may include one or more data items such as time of use, day of the week of use, date of use, amount of the water that was used for a given period, the unique ID of the shower unit (physical consumable provisioning device), and the unique ID of the user. The monitoring device monitors the detected usage data. The monitoring may be carried out in real time or maybe carried out at defined times or in defined time periods. The processor in the monitoring device detects abnormal usage of the water being provisioned by the shower unit by monitoring the detected usage data that it is receiving from the control device and utilising a process as described in further detail herein to analyse the usage data based on prior usage data in order to assess whether abnormal usage is occurring. The monitoring device may then generate an alert when the abnormal usage has been detected.

The generated alert may be in any suitable format. The alert is then communicated by the monitoring device to the central gateway device. The central gateway device then communicates the alert to the server. The server then communicates the alert to a registered device that has been registered with that particular electronic control device, monitoring device or a user associated with the electronic control device or monitoring device. For example, the registered device may be a mobile telephone that has been registered by an aged car facility or a health facility.

It will be understood that one or more of the monitoring device performs the entire detection process used to detect abnormal usage.

In accordance with any one of examples described herein, different analytical methods may be used by the monitoring device to carry out the detection of the abnormal usage.

The detected usage data may be compared with prior usage data associated with prior use of the physical consumable by the physical consumable provision device. For example, prior water usage data may be stored. Abnormal usage of water detected based on a comparison of the detected usage data with the prior usage data. For example, if the volume of water detected for a particular bathroom product exceeds a volume of water previously used in a defined period, then the system may flag this usage as an abnormal usage and generate an alert accordingly.

The processor in the device may compare the detected usage data with the prior usage data in order to detect whether the detected usage data matches, fits into or corresponds with a pattern associated with the prior usage data. Upon comparing, if the processor determines that the detected usage data does not match, deviates above a defined threshold or is substantially different to the pattern, then an alert may be generated.

The detected usage data may be compared with the prior usage data by detecting a maximum and/or minimum prior usage value in the prior usage data and then comparing the detected maximum and/or minimum prior usage value with maximum and/or minimum values in the detected usage data in a defined period. For example, a detected maximum prior usage value may be compared with an associated maximum value in the detected usage data. Alternatively, a detected minimum prior usage value may be compared with an associated minimum value in the detected usage data. Alternatively, a detected maximum prior usage value and a detected minimum prior usage value may be compared with an associated maximum value in the detected usage data and a minimum value in the detected usage data. In each of these examples, if the difference between the two compared values is outside of a determined threshold, or not within a determined threshold, an alert may be generated This detected usage data may be compared with the prior usage data based on an amount the physical consumable has been used (or not used) in a defined usage period.

A current usage amount of the physical consumable may be compared with a previously detected usage amount of the physical consumable. An alert may be generated if the current usage amount is determined to be below or above a defined threshold associated with the previously detected usage amount. This detected usage data (current usage amount) may be compared with the prior usage data based on a time that the physical consumable was or is being used in a defined usage period. That is, the current usage amount may be a quantitative value associated with a defined time or period.

A current usage time (e.g. a specific time of day or night, or a time period (e.g. morning, lunchtime, evening etc.) of the physical consumable may be compared with a previously detected usage time of the physical consumable in the usage period. An alert may be generated if the current usage time is below or above a defined threshold associated with the previously detected usage time.

In order to carry out the analytic steps, the prior usage data may be monitored and recorded.

Further, the abnormal usage detection process may analyse the usage data to determine whether the usage data matches any patterns known to be considered an abnormal use. Alternatively, the abnormal usage detection process may analyse the usage data to determine whether the usage data does not match any patterns known to be considered normal use. If the usage data is determined to differ from patterns known to be considered normal by a determined threshold, or usage data is determined to be similar enough to patterns known to be abnormal within a determined threshold, then the system may determine that abnormal usage has occurred and an alert or alarm may be generated.

A baseline of normal usage data may also be generated and stored. The abnormal usage detection process may analyse the usage data and compare it with the baseline data to determine whether the usage data is considered to be abnormal or normal use dependent on how much the usage data varies compared with the baseline data. If the variation exceeds or is below a determined threshold, then the system may determine that abnormal usage has occurred and an alert or alarm may be generated.

Therefore, the process and system described herein enables the detection of abnormal usage of water in a physical consumable usage environment, such as a bathroom. Detected usage data that is associated with the provision of water is used to detect abnormal usage of water based on the monitoring of the detected usage data.

In the examples described above, the bathroom is an example of a physical consumable usage environment. It will be understood that the methods and systems described herein may be applied to other water usage environments. The detection of abnormal usage may then be used to generate an alert or alarm.

It will also be understood that the physical consumable monitoring devices described herein are used to monitor water.

It will be understood that the detection or determination of abnormal usage may include the detection or determination of non-usage of water as well as usage of water, where that non-usage and usage are determined to be abnormal.

### Industrial Applicability

The arrangements described are applicable to the consumable provisioning industries, data analytics industries and particularly for industries concerned with detecting whether a person is incapacitated or requires assistance.

The foregoing describes only some embodiments of the present invention, and modifications and/or changes can be made thereto without departing from the scope of the invention, the embodiments being illustrative and not restrictive.

In the context of this specification, the word "comprising" means "including principally but not necessarily solely" or "having" or "including", and not "consisting only of". Variations of the word "comprising", such as "comprise" and "comprises" have correspondingly varied meanings.

## Claims

1. A method for detecting abnormal usage of water in a water usage environment (107), **characterized in that** the method comprises the steps of:
monitoring detected usage data associated with the provision of water in a water usage environment (107) using at least one water provisioning device (101, 103, 105) that is arranged to provide the water; and using a water monitoring device (201) which is part of or integrated with one of the at least one water provisioning device (101, 103, 105); and
detecting abnormal usage of the water based on the monitoring of the detected usage data, wherein the detecting of the abnormal usage is performed by the water monitoring device (201) associated with the water provisioning device (101, 103, 105), wherein the method further comprising the steps of comparing the detected usage data with prior usage data associated with prior use of the water by the water provisioning device (101, 103, 105), and detecting the abnormal usage of the water based on the comparison of the detected usage data with the prior usage data, wherein the step of comparing the detected usage data with the prior usage data comprises detecting whether the detected usage data matches a usage pattern associated with the prior usage data or wherein the step of comparing the detected usage data with the prior usage data comprises detecting a maximum and/or minimum prior usage value in the prior usage data and comparing the detected maximum and/or minimum prior usage value with maximum and/or minimum values in the detected usage data.

2. The method of claim 1 further comprising the step of generating an alert based on the detection of the abnormal usage and further comprising the step of sending the generated alert to one or more registered devices (115) associated with the usage of the water.

3. A water monitoring device (201) comprising I/O port (209), transceiver module (211), a power unit (203), a processor (205) and memory (207), wherein the I/O port (209) connects to a sensor that senses usage of water (213), **characterized in that** the water monitoring device is part of or integrated with a water provisioning device arranged to provide water and **in that** the memory (207) comprises software code arranged to cause the processor (205) to operate according to defined instructions to implement the method according to claim 1.

4. The water monitoring device (201) of claim 3, wherein the water monitoring device (201) is part of or integrated with one or more of an item of tap ware (101), a shower unit (103) and a flush unit (105).

5. A building management system comprising one or more water monitoring devices according to claim 3 and a central gateway device, wherein the central gateway device (109) comprises a power unit (303), a transceiver module (311), a processor (305) and memory (307), **characterized in that** the transceiver module (311) connects and communicates data with one or more water monitoring devices (201) according to claim 3.

6. A system comprising a management building system according to claim 5 and a server (111) in communication with the building management system, wherein the server (111) comprises a power unit (403), an input module (413), an output module (415), a processor (405) and memory (407), **characterized in that** the input module (413) of the server (111) connects and receives data from the central gateway device (109).

## Patentansprüche

1. Verfahren zur Erkennung einer ungewöhnlichen Wassernutzung in einer Wassernutzungsumgebung (107), **dadurch gekennzeichnet, dass** das Verfahren die folgenden Schritte aufweist:
Überwachen detektierter Nutzungsdaten im Zusammenhang mit der Bereitstellung von Wasser in einer Wassernutzungsumgebung (107) unter Verwendung mindestens einer Wasserbereitstellungseinrichtung (101, 103, 105), die zur Bereitstellung von Wasser angeordnet ist; und Verwenden einer Wasserüberwachungsvorrichtung (201), die Teil von einer der wenigstens einen Wasserbereitstellungsvorrichtung ist oder in dieser integriert ist, und
Erkennen einer ungewöhnlichen Wassernutzung basierend auf der Überwachung der detektierten Nutzungsdaten, wobei das Detektieren der ungewöhnlichen Nutzung durch die der Wasserbereitstellungsvorrichtung (101, 103, 105) zugeordneten Wasserüberwachungsvorrichtung (201) erfolgt, wobei das Verfahren ferner die weiteren Schritte Vergleichen der detektierten Nutzungsdaten mit vorherigen Nutzungsdaten einer vorherigen Wassernutzung durch die Wasserbereitstellungsvorrichtung (101, 103, 105) und Detektieren der ungewöhnlichen Wassernutzung auf Basis des Vergleichs von detektierten Nutzungsdaten mit vorherigen Nutzungsdaten aufweist, wobei der Schritt des Vergleichens der detektierten Nutzungsdaten mit den vorherigen Nutzungsdaten das Detektieren, ob die detektierten Nutzungsdaten einem Nutzungsprofil der vorherigen Nutzung entsprechen, aufweist, oder wobei der Schritt des Vergleichens der detektierten Nutzungsdaten mit den vorherigen Nutzungsdaten das Detektieren eines maximalen und/oder minimalen vorherigen Nutzungswertes in den vorherigen Nutzungsdaten und Vergleichen des detektierten maximalen und/oder minimalen vorherigen Nutzungswertes mit Maximal- und/oder Minimalwerten in den detektierten Nutzungsdaten aufweist.

2. Verfahren nach Anspruch 1, das weiterhin den Schritt Erzeugen einer Benachrichtigung auf Grundlage der Detektion der ungewöhnlichen Nutzung sowie ferner den Schritt Versenden der erzeugten Benachrichtigung an eine oder mehrere der Wassernutzung zugeordnete registrierte Vorrichtungen (115) aufweist.

3. Wasserüberwachungsvorrichtung (201) mit einem I/O-Port (209), einem Transceiver-Modul (211), einer Stromversorgung (203), einem Prozessor (205) und einen Speicher (207), wobei der I/O-Port (209) mit einem Wassernutzungserkennungssensor (213) verbunden ist, **dadurch gekennzeichnet, dass** die Wasserüberwachungsvorrichtung (201) Teil einer Wasserbereitstellungseinrichtung (101, 103, 105), die zur Bereitstellung von Wasser vorgesehen ist, ist oder in diese integriert ist, und dass der Speicher (207) einen Softwarecode aufweist, durch den der Prozessor nach definierten Instruktionen arbeitet, um das Verfahren nach Anspruch 1 durchzuführen.

4. Wasserüberwachungsvorrichtung (201) nach Anspruch 3, wobei die Wasserüberwachungsvorrichtung (201) in einem oder mehreren Gegenstände, wie einer Armatur (101), einer Duscheinheit (103) und einer Spülungseinheit (105), Teil ist oder darin integriert ist.

5. Gebäudemanagementsystem, welches eine oder mehrere der Wasserüberwachungsvorrichtungen (201) nach Anspruch 3 und eine zentrale Gateway-Vorrichtung (109) aufweist, wobei die zentrale Gateway-Vorrichtung (109) eine Stromversorgung (303), ein Transceiver-Modul (311), einen Prozessor (305) und Speicher (307) aufweist, **dadurch gekennzeichnet, dass** das Transceiver-Modul (311) mit einer oder mehreren Wasserüberwachungsvorrichtungen (201) nach Anspruch 3 verbunden ist und Daten austauscht.

6. System mit einem Gebäudemanagementsystem nach Anspruch 5 und einem Server (111) in Kommunikation mit dem Gebäudemanagementsystem, wobei der Server (111) eine Stromversorgung (403), ein Eingabemodul (413), ein Ausgabemodul (415), einen Prozessor (405) und Speicher (407) aufweist, **dadurch gekennzeichnet, dass** das Eingabemodul (413) des Servers (111) mit der zentralen Gateway-Vorrichtung (109) verbunden ist und von dieser Daten empfängt.

## Revendications

1. Procédé pour détecter une utilisation anormale de l'eau dans un environnement d'utilisation d'eau (107), **caractérisé en ce que** le procédé comprend les étapes suivantes :
surveiller les données d'utilisation détectées associées à la fourniture de l'eau dans un environnement d'utilisation d'eau (107) en utilisant au moins un dispositif de fourniture de l'eau (101, 103, 105) qui est agencé pour fournir de l'eau ; et en utilisant un dispositif pour surveiller l'eau (201) qui est part de ou est intégré dans un de l'au moins un dispositif de fourniture de l'eau (101, 103, 105) ; et
détecter une utilisation anormale de l'eau sur la base de la surveillance des données d'utilisation détectées, la détection de l'utilisation anormale s'effectuant par le dispositif pour surveiller l'eau (201) associé au dispositif de fourniture de l'eau (101, 103, 105), le procédé comprenant en plus les étapes de comparer les données d'utilisation détectées avec des données d'utilisation antérieures d'une utilisation antérieure du dispositif de fourniture de l'eau (101, 103, 105) et détecter l'utilisation anormale sur la base de la comparaison des données d'utilisation détectées avec des données d'utilisation antérieures, l'étape de comparer les données d'utilisation détectées avec des données d'utilisation antérieures comprenant la détection, si les données d'utilisation détectées correspondent à un profil d'utilisation de l'utilisation antérieure, ou l'étape de comparer les données d'utilisation détectées avec des données d'utilisation antérieures comprenant la détection d'un valeur maximum et/ou minimum d'utilisation antérieure dans les données d'utilisation antérieures et la comparaison du valeur maximum et/ou minimum d'utilisation antérieure détecté avec des valeurs maximum et/ou minimum dans les données d'utilisation détectées.

2. Procédé selon la revendication 1 comprenant en plus l'étape de générer une alerte sur la base de détection de l'utilisation anormale et comprenant en outre l'étape d'envoyer l'alerte générée à un ou plusieurs dispositifs enregistrés (115) associés à l'utilisation de l'eau.

3. Dispositif pour surveiller l'eau (201) comprenant un port I/O (209), un module émetteur-récepteur (211), une unité d'alimentation (203), un processeur (205) et mémoire (207), le port I/O (209) étant connecté à un détecteur pour détecter une utilisation d'eau (213), **caractérisé en ce que** le dispositif pour surveiller l'eau (201) est part de ou est intégré dans un dispositif de fourniture de l'eau (101, 103, 105), qui est agencé pour fournir de l'eau, et **en ce que** la mémoire (207) comprend un code logiciel agencé pour amener le processeur (205) à mettre en oeuvre le procédé selon la revendication 1.

4. Dispositif pour surveiller l'eau (201) selon la revendication 3, le dispositif pour surveiller l'eau (201) étant part de ou étant intégré dans un ou plusieurs des unités de robinetterie (101), une unité de douche (103) et une unité de chasse d'eau (105).

5. Système d'entretien des bâtiments comprenant un ou plusieurs des dispositifs pour surveiller l'eau (201) selon la revendication 3 et un dispositif de passerelle centrale (109), le dispositif de passerelle centrale (109) comprenant une unité d'alimentation (303), un module émetteur-récepteur (311), un processeur (305) et mémoire (307), **caractérisé en ce que** le module émetteur-récepteur (311) est connecté à un ou plusieurs des dispositifs pour surveiller l'eau (201) selon la revendication 3 et communique des données avec le même.

6. Système avec un système d'entretien des bâtiments selon la revendication 5 et un serveur (111) qui communique avec le système des bâtiments, le serveur (111) comprenant une unité d'alimentation (403), une unité d'entrée (413), une unité de sortie (415), un processeur (405) et mémoire (407), **caractérisé en ce que** l'unité d'entrée (413) du serveur (111) est connecté au dispositif de passerelle centrale (109) et reçoit des données du même.
